# EUROPÄISCHE PATENTSCHRIFT

(11) **EP 1 795 230 B1**
(45) Veröffentlichungstag und Bekanntmachung des Hinweises auf die Patenterteilung: **04.03.2009**
(21) Anmeldenummer: 06124750.8
(22) Anmeldetag: 24.11.2006
(51) Int. Cl.: A61N 5/10

(54) **Medizinische Bestrahlungseinrichtung**
Medical irradiation device
Dispositif médicale de rayonnement

(30) Priorität: 09.12.2005 DE 102005058871; 09.12.2005 US 748849 P
(43) Veröffentlichungstag der Anmeldung: 13.06.2007
(73) Patentinhaber: SIEMENS AKTIENGESELLSCHAFT, 80333 München (DE)
(72) Erfinder: Rietzel, Eike, 64289 Darmstadt (DE)

(56) Entgegenhaltungen:
- WO-A-98/10696
- WO-A-03/039370
- WO-A-20/04047923
- WU QIUWEN ET AL: "Algorithms and functionality of an intensity modulated radiotherapy optimization system" MEDICAL PHYSICS, AIP, MELVILLE, NY, US, Bd. 27, Nr. 4, April 2000 (2000-04), Seiten 701-711, XP012011107 ISSN: 0094-2405

## Beschreibung

Die Erfindung betrifft eine medizinische Bestrahlungseinrichtung sowie ein Verfahren zur Einstellung von Betriebsparametern einer medizinischen Bestrahlungseinrichtung.

Eine medizinische Bestrahlungseinrichtung ist beispielsweise aus der DE 35 02 776 A1 bekannt. Diese Bestrahlungseinrichtung umfasst einen Linearbeschleuniger als Strahlenquelle sowie einen so genannten Simulator mit Röntgenröhre für eine präzise Positionierung des Patienten.

Aus der DE 100 25 913 A1 ist eine mit Partikelstrahlen, nämlich Ionenstrahlen, arbeitende Bestrahlungseinrichtung bekannt, wobei vorgesehen ist, in einem Bestrahlungsplan die Energie sowie die Dosis der Ionen festzulegen und damit die Eindringtiefe der Ionenstrahls und die Bestrahlungsmenge zu bestimmen. Zur Überwachung der Bestrahlung ist im Bestrahlungsraum eine PET-Kamera installiert.

Die WO 03/039370 A1 offenbart eine Vorrichtung, die zum Anzeigen, Führen und Abzielen einer externen Strahlentherapie vorgesehen ist. Bei der Behandlung eines Weichteilkarzinoms, insbesondere eines Prostatakarzinoms, wird zur Beobachtung in Echtzeit eine Ultraschallsonde verwendet, welche eine ausreichende Bildqualität in einem kostengünstigen medizintechnischen System liefern soll. Des Weiteren werden in dem bekannten System Röntgen/CT-Daten verwendet, die mit den Ultraschalldaten zusammengeführt werden.

Ein von Wu Qiuwen et al. verfasster Artikel: "Algorithms and functionality of an intensity modulated radiotherapy optimization system" (Medical Physics, AIP, Melville, NY, US, Bd. 27, Nr. 4, April 2000, S. 701-711, XP012011107 ISSN: 0094-2405) hat Verfahren zum Gegenstand, mit denen die Möglichkeiten, welche herkömmliche Bestrahlungs-Planungs-Systeme bieten, besser ausgeschöpft werden sollen. Hierbei werden Algorithmen angewandt, die mit zahlreichen, zum Teil hunderten, Iterationen zu einer Optimierung der Planung führen sollen.

Die WO 98/10696 A1 offenbart ein Megavolt Radiotherapiegerät mit einer diagnostischen Bilderzeugungsvorrichtung. Hierbei befindet sich in einem ersten Raum das Radiotherapiegerät, beispielsweise ein Linearbeschleuniger, während ein bildgebendes Diagnosegerät, insbesondere ein Computertomographie- oder ein Magnetresonanzgerät, in einem anderen Raum angeordnet ist. Es ist vorgesehen, einen Patienten unter Beibehaltung einer definierten Positionierung zwischen dem Diagnose- und dem Therapiegerät zu transportieren.

Aus der WO 2004/047923 A1 ist ein Strahlentherapiegerät bekannt, welches eine Strahlenquelle für therapeutische Strahlung, eine Strahlenquelle für diagnostische Strahlung, sowie eine Datenverarbeitungsvorrichtung umfasst, die insbesondere für die Verarbeitung von Schnittbildern vorgesehen ist. Hierbei sind mehrere Schnittbilder vorzugsweise orthogonal zueinander angeordnet.

Bei medizinischen Bestrahlungseinrichtungen wird allgemein angestrebt, ausschließlich zu behandelndes Gewebe der Strahlung auszusetzen und umgebendes Gewebe weitestmöglich zu schonen. Dieses Ziel ist mit Partikelstrahlung aufgrund des in diesem Fall gegebenen inversen Dosisprofils mit besonders guter Näherung erreichbar. Im Gegensatz zu elektromagnetischer Strahlung geben Partikel typischerweise am Ende ihrer Reichweite am meisten Energie ab. Diese Eigenschaft von Partikelstrahlen erweitert die Möglichkeiten der Behandlung von Tumoren, die an Risikoorgane grenzen. In einem solchen Fall ist eine präzise Erstellung und Einhaltung eines Bestrahlungsplanes von besonderer Bedeutung. Hierbei ist zu berücksichtigen, dass sich Form und Dichte des bestrahlten Gewebes im Laufe der Strahlentherapie, insbesondere durch die Wirkung der Bestrahlung, stark ändern können. Es kann daher erforderlich werden, während der Strahlentherapie einen neuen Bestrahlungsplan zu erstellen.

Der Erfindung liegt die Aufgabe zugrunde, eine insbesondere mit Partikelstrahlung arbeitende medizinische Bestrahlungseinrichtung anzugeben, welche besonders dazu geeignet ist, die Bestrahlung an sich im Laufe der Therapie ändernde Bedingungen anzupassen.

Diese Aufgabe wirderfindungsgemäß gelöst durch eine medizinische Bestrahlungseinrichtung mit den Merkmalen des Anspruchs 1. Sofern im Folgenden therapeutische Verfahren offenbart sind, haben die entsprechenden Passagen lediglich erläuternden Charakter.

Die Bestrahlungseinrichtung umfasst eine Strahlenquelle, insbesondere eine Partikelstrahlenquelle, und eine Ablenkeinrichtung, worunter jegliche Einrichtung verstanden wird, die die Strahlung, insbesondere die Energie und Richtung der Teilchen, steuerbar beeinflusst. Zur Ansteuerung der Bestrahlungseinrichtung ist eine Datenverarbeitungsvorrichtung vorgesehen, welche Betriebsparameter der Strahlenquelle und der Ablenkeinheit entsprechend einem Bestrahlungsplan einstellt. Der Bestrahlungsplan wurde zuvor auf Grundlage einer mit einem bildgebenden medizintechnischen Diagnosegerät, insbesondere Computertomographie-Gerät, erstellten Aufnahme des zu bestrahlenden Gewebes generiert. Die Datenverarbeitungsvorrichtung ist programmtechnisch derart eingerichtet, dass mehrere Charakteristika der auf das Gewebe einwirkenden Strahlung in einer gemeinsamen Darstellung visualisierbar sind. Die Charakteristika werden dabei anhand unterschiedlicher Bestrahlungsszenarien für zeitlich aufeinanderfolgende Bestrahlungen ermittelt. Die Darstellung umfasst insbesondere ein vergangenheitsbezogenes Bestrahlungsszenario und ein zukunftsbezogenes Bestrahlungsszenario. Insbesondere werden hierbei Bestrahlungsszenarien unter Berücksichtigung der gleichen Bestrahlungsart und bevorzugt der gleichen Strahlungsquelle ermittelt. Bei den mit Hilfe der Datenverarbeitungsvorrichtung darstellbaren Charakteristika der Strahlung handelt es sich insbesondere um die im Gewebe deponierte Energie. Ebenso ist die Reichweite der Strahlung darstellbar. Die graphische Darstellung von Eigenschaften der gemäß Bestrahlungsplan zu applizierenden Strahlung ist in vorteilhafter Weise eingebunden in eine zwei- oder dreidimensionale Darstellung des zu bestrahlenden Gewebes.

Im erstgenannten Bestrahlungsszenario wird ausgegangen von einer ersten mit dem bildgebenden medizintechnischen Diagnosegerät erstellten Aufnahme des zu bestrahlenden Gewebes, welche vor Beginn der Strahlentherapie gewonnen wurde. Auf Grundlage dieser Aufnahme wurde rechnergestützt ein erster Bestrahlungsplan generiert. Die diesem Bestrahlungsplan entsprechenden Betriebsparameter der medizinischen Bestrahlungseinrichtung werden zunächst nicht geändert. Vielmehr wird mit diesen Betriebsparametern ein zweites Bestrahlungsszenario betrachtet, das nicht von der ursprünglichen Aufnahme des zu bestrahlenden Gewebes ausgeht, sondern von einer neueren, nach Bestrahlung des Gewebes gewonnenen Aufnahme.

Aufgrund der geänderten Geometrie und/oder Zusammensetzung des zu bestrahlenden Gewebes ergibt sich eine geänderte Charakteristik, insbesondere Reichweite, der Strahlung. Diese Charakteristik wird rechnergestützt in eine Darstellung der ursprünglichen Charakteristik eingeblendet. In diesem Schritt wird somit ein zweiter Bestrahlungsplan simuliert, wobei sich zunächst nur die Form und/oder Art des bestrahlten Gewebes ändert. Der die Bestrahlungseinrichtung einschließlich Datenverarbeitungsanlage nutzende Bediener wird durch das zweite Bestrahlungsszenario in die Lage versetzt, sich ein Bild von den Wirkungen der Strahlung unter den geänderten Bedingungen zu machen, wobei die geänderten Wirkungen visuell unmittelbar den Wirkungen laut ursprünglichem Bestrahlungsplan gegenübergestellt werden.

In einem nächsten Schritt hat der Bediener die Möglichkeit, ein Szenario mit geänderten Parametereinstellungen der Bestrahlungseinrichtung zu simulieren. Hierbei ist es besonders vorteilhaft, dass der Bediener von geometrisch darstellbaren Charakteristiken der Bestrahlung, insbesondere Reichweiten und/oder Dosisverteilungen, ausgehen kann. Aus vom Benutzer vorgebbaren geänderten Charakteristiken ermittelt die Datenverarbeitungsanlage geeignete neue Betriebsparameter der Bestrahlungseinrichtung. Die Bestrahlung ist im folgenden mit der neuen Parametereinstellung simulierbar, wobei die Resultate wiederum, den Resultaten einer bereits durchgeführten Bestrahlung und/oder eines anderen Szenarios gegenübergestellt, angezeigt werden können. Dem Bediener wird damit eine sehr rasche sowie sachgerechte Anpassung des Bestrahlungsplans an geänderte Gegebenheiten ermöglicht.

Nach einer bevorzugten Ausgestaltung ist eine Visualisierung der Charakteristika der Strahlung unter Nutzung des volume rendering (VR) Verfahrens und/oder des surface rendering (SR) Verfahrens vorgesehen. Das VR Verfahren ermöglicht eine semitransparente Darstellung von Strukturen und ist beispielsweise in der Firmenschrift "electro medica", Heft 1, 2003, Seiten 50 - 57, der Siemens AG beschrieben. Weitere Informationen, auch zum SR Verfahren, enthält die Dissertation "Dreidimensionale Darstellung der Hirnnerven V-VIII mittels virtueller Zisternoskopie" (Christian Nikolaus Heine, Medizinische Fakultät der Charite - Universitätsmedizin Berlin, 2004). Mit Hilfe des VR Verfahrens sind Abweichungen zwischen verschiedenen Bestrahlungsszenarien dreidimensional über das gesamte relevante Volumen darstellbar. Zusätzlich oder alternativ ist auch eine schichtweise vergleichende Darstellung möglich.

Die Erfindung hat insbesondere den Vorteil, dass mit Hilfe einer vergleichenden Visualisierung, die unterschiedliche Bestrahlungsszenarien abdeckt, eine sehr schnelle und für den Bediener unkomplizierte Einstellung von Parametern eines Bestrahlungsplanes möglich ist.

Nachfolgend wird ein Ausführungsbeispiel der Erfindung anhand einer Zeichnung näher erläutert. Hierin zeigen jeweils in vereinfachter Darstellung:
- FIG 1: eine medizinische Bestrahlungseinrichtung sowie ein mit dieser datentechnisch verknüpftes bildgebendes medizintechnisches Diagnosegerät,
- FIG 2: ein mit der medizinischen Bestrahlungseinrichtung nach FIG 1 zu bestrahlendes Volumen, und
- FIG 3: in einem Flussdiagramm ein Verfahren zur Einstellung von Betriebsparametern der medizinischen Bestrahlungseinrichtung nach FIG 1.

Einander entsprechende Teile oder Parameter sind in allen Figuren mit den gleichen Bezugszeichen gekennzeichnet.

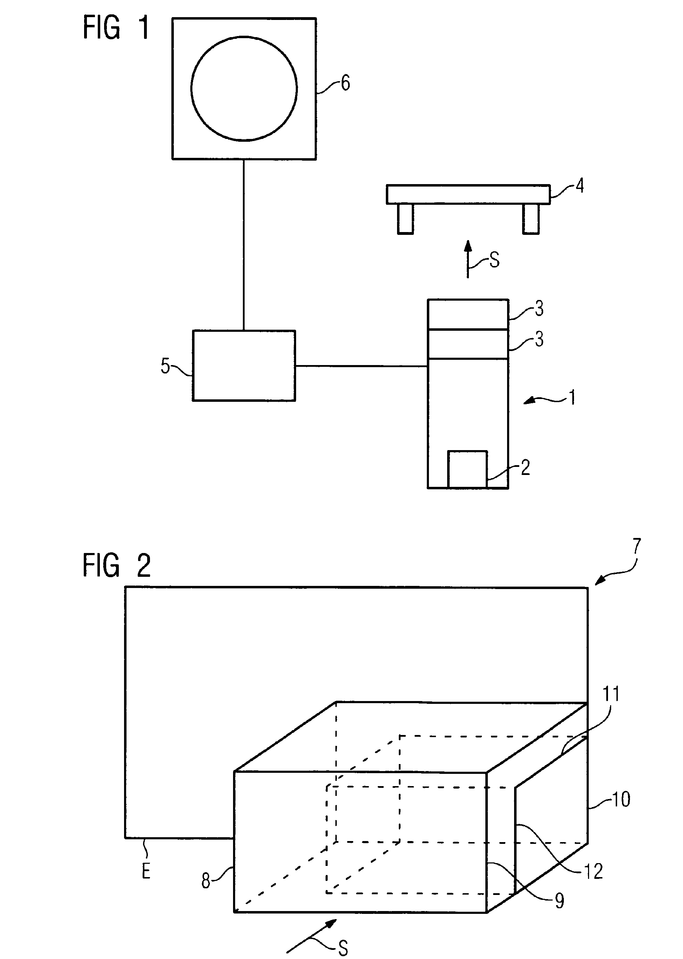

Die FIG 1 zeigt in symbolisierter Darstellung eine medizinische Bestrahlungseinrichtung 1, welche eine Strahlenquelle 2, nämlich Partikelstrahlenquelle, sowie eine allgemein als Ablenkeinrichtung 3 bezeichnete Einrichtung zur Beeinflussung der Energie und/oder Richtung der Strahlung umfasst. Die Strahlrichtung des Partikelstrahls ist mit S angegeben. Ein nicht dargestellter Patient mit einem zu bestrahlenden Gewebe befindet sich auf einer Patientenliege 4. Die Bestrahlung erfolgt gemäß eines Bestrahlungsplanes, der Parametereinstellungen der Strahlenquelle 2 sowie der Ablenkeinrichtung 3 und damit zusammenhängend Parameter der Partikelbestrahlung wie Strahlgeometrie, Energie der Partikel und Dosis der Bestrahlung vorgibt. Die Speicherung der zur Umsetzung des Bestrahlungsplans erforderlichen Daten sowie die Ansteuerung der Strahlenquelle 2 und der Ablenkeinrichtung 3 erfolgt mittels einer Datenverarbeitungsvorrichtung 5, die datentechnisch auch mit einem bildgebenden medizintechnischen Diagnosegerät 6, nämlich einem Computertomographen, verbunden ist. Alternativ kann es sich bei dem bildgebenden medizintechnischen Diagnosegerät 6 beispielsweise um ein Magnetresonanzgerät handeln.

Eine mit dem bildgebenden medizintechnischen Diagnosegerät 6 gewonnene Aufnahme des zu bestrahlenden Gewebes sowie umgebender Gewebebereiche des Patienten dient als Grundlage zur Erstellung des Bestrahlungsplans. Die Datenverarbeitungsvorrichtung 5 ermöglicht eine dreidimensionale Darstellung des zu bestrahlenden Gewebes.

Die FIG 2 zeigt eine grob schematisierte, mittels der Datenverarbeitungsvorrichtung 5 wiedergebbare Darstellung 7 möglicher Formen eines zu bestrahlenden Volumens. Ein in der Darstellung quaderförmiges erstes Volumen 8 ist laut Bestrahlungsplan der in Strahlrichtung S eintreffenden Partikelstrahlung ausgesetzt. Dieses erste Volumen 8 entspreche der mit Hilfe des bildgebenden medizintechnischen Diagnosegerätes 6 festgestellten Gestalt eines Tumors. Die minimale und maximale Energie der von der Strahlenquelle 2 emittierten Partikel ist derart bemessen, dass die Teilchen mit bestmöglicher Näherung ausschließlich in dem durch eine proximale Grenzfläche 9 und eine distale Grenzfläche 10 begrenzten ersten Volumen 8 ihre Energie abgeben. In der Darstellung 7 kann in einer dreidimensionalen Ansicht auch die Dosisverteilung der mittels der Strahlenquelle 2 deponierten Strahlung angezeigt werden. Hierbei werden unterschiedliche Dosen beispielsweise durch verschiedene Farben angezeigt.

Eine erhöhte Übersichtlichkeit ist gegeben, wenn in der Darstellung 7 nur Grenzflächen, insbesondere die proximale Grenzfläche 9 und die distale Grenzfläche 10 visualisiert werden. Fallen die Grenzflächen 9,10 genau mit Oberflächen des zu bestrahlenden Gewebes zusammen, was idealerweise der Fall sein sollte, so ergibt sich die in FIG 2 gezeigte Ansicht. Hierfür sind das volume rendering Verfahren und das surface rendering Verfahren besonders geeignet. Beide Verfahren stellen Rekonstruktionstechniken dar, wobei nach dem surface rendering Verfahren in der Regel weniger als 10 % der Bilddaten genutzt werden, während das volume rendering Verfahren nahezu alle Daten nutzt und mit wesentlich geringerer Wahrscheinlichkeit Artefakte bildet. Die in der Darstellung 7 sichtbaren Grenzflächen 9,10 entsprechen der sogenannten proximalen beziehungsweise distalen Kante der Partikelstrahlung.

Durch die Bestrahlung des Tumors verringert sich im Laufe der Zeit dessen Volumen, was mit Hilfe des Diagnosegerätes 6 diagnostizierbar ist. Die Ausdehnung des Tumors, welcher sich zurückgebildet hat, entspricht einem zweiten Volumen 11, das in der Darstellung 7 gestrichelt eingezeichnet ist. Während die Lage der distalen Grenzfläche 10 im Vergleich zum ersten Volumen 8 unverändert, nämlich in einer Ebene E liegend, ist, weist das zweite Volumen 11 eine in Strahlrichtung S verschobene proximale Grenzfläche 12 auf. Diese Verschiebung ist in einem geänderten, dem verkleinerten Tumor angepassten Bestrahlungsplan zu berücksichtigen. Der Bediener der Bestrahlungseinrichtung 1 einschließlich der Datenverarbeitungsvorrichtung 5 hat die Möglichkeit, Charakteristika, insbesondere Dosisverteilungen sowie die Lage von Grenzflächen 9,10,12, der Bestrahlung simulieren zu lassen.

Wird beispielsweise bei einer Simulation davon ausgegangen, dass ausschließlich das zweite Volumen 11 zu bestrahlen ist, wobei die Parametereinstellungen des ursprünglichen Bestrahlungsplans beibehalten werden, so würden in der Darstellung 7 Grenzflächen der Bestrahlung eingeblendet werden, die deutlich außerhalb des zweiten Volumens 11 liegen. Für den Bediener wäre dies ein klarer Hinweis, dass die Parametereinstellungen geändert werden müssen. Mit angepassten Parametereinstellungen kann anschließend die Simulation erneut durchgeführt werden. In vorteilhafter Weise ist die Datenverarbeitungsvorrichtung 5 programmtechnisch dazu eingerichtet, bei vorgegebenem zu bestrahlenden Volumen 8,11 eine für die Partikelbestrahlung geeignete Parametereinstellung der Strahlenquelle 2 sowie der Ablenkeinrichtung 3 selbsttätig zu finden.

In analoger Weise zum ersten Volumen 8 werden auch im Fall des zweiten Volumens 11 nicht nur Oberflächen des Volumens 8 selbst, sondern auch Grenzflächen 10,12 der Bestrahlung automatisch in die dreidimensionale Darstellung 7 eingefügt. Im idealisierten Ausführungsbeispiel nach FIG 2 fallen auch beim zweiten, kleineren Volumen 11 die Grenzflächen 10,12 exakt mit Oberflächen des Volumens 11 zusammen. Allgemein wird die Simulation automatisch für jede Strahlrichtung S, das heißt für jede Einschussbahn durchgeführt.

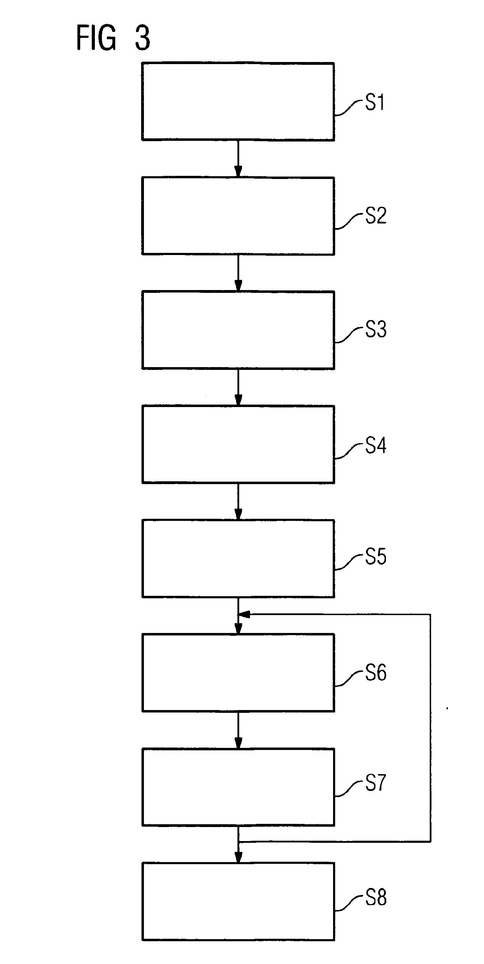

Der Ablauf der Einstellung und Anpassung von Betriebsparametern der Bestrahlungseinrichtung 1 wird im Folgenden anhand FIG 3 erläutert:
In einem ersten Schritt S1 wird mittels des bildgebenden medizintechnischen Diagnosegerätes 6 eine dreidimensionale Aufnahme des zu bestrahlenden Gewebes erstellt. Im zweiten Schritt S2 wird auf Basis dieser Aufnahme rechnergestützt ein erster Bestrahlungsplan generiert. Die Bestrahlung wird im dritten Schritt S3 entsprechend diesem Plan durchgeführt. Zwischen zwei als Fraktionen bezeichneten Bestrahlungen wird in einem vierten Schritt S4 abermals eine Aufnahme des zu bestrahlenden Gewebes mit Hilfe des Diagnosegerätes 6 erstellt. Diese zweite Aufnahme (siehe zweites Volumen 11 in FIG 2) wird herangezogen, um in einem fünften Schritt S5 in einer Simulation die Resultate eines zweiten Bestrahlungsplans zu simulieren, in dem zunächst die Parametereinstellungen der Bestrahlungseinrichtung 1 unverändert bleiben, jedoch die diagnostizierten Änderungen des zu bestrahlenden Gewebes berücksichtigt sind. Im folgenden sechsten Schritt S6 werden die Ergebnisse der Simulation, das heißt Charakteristika der gemäß der verschiedenen Szenarien durchgeführten beziehungsweise simulierten Bestrahlung, simultan in einer gemeinsamen Darstellung 7 (FIG 2), welche einen direkten Vergleich der verschiedenen Resultate ermöglicht, dargestellt. Im nächsten Schritt S7 werden die Parametereinstellungen der Bestrahlungseinrichtung 1 in einer erneuten Simulation geändert, um die Ergebnisse wiederum in einer Darstellung 7 (Schritt S6) anzuzeigen. Dieser Vorgang wird, soweit erforderlich, mehrmals wiederholt, bis im Schritt S8 die Bestrahlung nach einem aktualisierten Bestrahlungsplan fortgeführt wird, womit die Konformität der Bestrahlung gewahrt bleibt.

## Patentansprüche

1. Medizinische Bestrahlungseinrichtung, mit einer Strahlenquelle (2), einer Ablenkeinrichtung (3) und einer Datenverarbeitungsvorrichtung (5), wobei die Strahlenquelle (2) und die Ablenkeinrichtung (3) entsprechend einem unter Nutzung einer mit einem bildgebenden medizintechnischen Diagnosegerät (6) erstellten Aufnahme eines zu bestrahlenden Gewebes generierten Bestrahlungsplan mittels der Datenverarbeitungsvorrichtung (5) ansteuerbar sind,
**dadurch gekennzeichnet, dass** die Datenverarbeitungsvorrichtung (5) datentechnisch derart eingerichtet ist, dass Charakteristika der auf das Gewebe einwirkenden Strahlung in einer gemeinsamen Darstellung (7) visualisierbar sind, wobei die Charakteristika auf Grundlage zumindest zweier unterschiedlicher Bestrahlungspläne, nämlich eines vergangenheitsbezogenen Bestrahlungsplanes und eines zukunftsbezogenen Bestrahlungsplanes, ermittelt sind, und wobei die verschiedenen Bestrahlungspläne unterschiedliche Geometrien des zu bestrahlenden Gewebes umfassen.

2. Medizinische Bestrahlungseinrichtung nach Anspruch 1,
**dadurch gekennzeichnet, dass** die Strahlenquelle (2) eine Partikelstrahlenquelle ist.

3. Medizinische Bestrahlungseinrichtung nach Anspruch 1 oder 2,
**dadurch gekennzeichnet, dass** in den verschiedenen Bestrahlungsplänen eine zeitliche Veränderung der Geometrie des zu bestrahlenden Gewebes berücksichtigt ist.

4. Medizinische Bestrahlungseinrichtung nach einem der Ansprüche 1 bis 3,
**dadurch gekennzeichnet, dass** visualisierbare Charakteristika der auf das Gewebe einwirkenden Strahlung Dosisverteilungen sind.

5. Medizinische Bestrahlungseinrichtung nach einem der Ansprüche 1 bis 4,
**dadurch gekennzeichnet, dass** Charakteristika der auf das Gewebe einwirkenden Strahlung zusammen mit dem zu bestrahlenden Gewebe in einer Darstellung (7) visualisiert sind.

6. Medizinische Bestrahlungseinrichtung nach einem der Ansprüche 1 bis 5,
**dadurch gekennzeichnet, dass** die Darstellung (7) die Visualisierung der relevanten geometrischen Reichweiten der auf das Gewebe einwirkenden Strahlung umfasst.

7. Medizinische Bestrahlungseinrichtung nach einem der Ansprüche 1 bis 6,
**dadurch gekennzeichnet, dass** eine Visualisierung der Charakteristika der Strahlung unter Nutzung des volume rendering Verfahrens vorgesehen ist.

8. Medizinische Bestrahlungseinrichtung nach einem der Ansprüche 1 bis 7,
**dadurch gekennzeichnet, dass** eine Visualisierung der Charakteristika der Strahlung unter Nutzung des surface rendering Verfahrens vorgesehen ist.

9. Medizinische Bestrahlungseinrichtung nach einem der Ansprüche 1 bis 8,
**dadurch gekennzeichnet, dass** eine schichtweise Visualisierung der Charakteristika der Strahlung vorgesehen ist.

## Claims

1. Medical radiation apparatus, with a beam source (2), a deflection apparatus (3), and a data processing device (5), with the beam source (2) and the deflection apparatus (3) being activatable by means of the data processing device (5) according to a radiation scenario generated using a recording of tissue to be irradiated produced using a medical imaging diagnosis device (6),
**characterised in that**
the data processing device (5) is set up for data purposes such that characteristics of the radiation acting on the tissue can be visualised in a common display (7) with the characteristics being determined on the basis of at least two different radiation scenarios, namely a past-related radiation scenario and a future-related radiation scenario and with the different radiation scenarios comprising different geometries of the tissue to be radiated.

2. Medical radiation apparatus according to claim 1,
**characterised in that**
the beam source (2) is a particle beam source.

3. Medical radiation apparatus according to claim 1 or 2,
**characterised in that**
a change over time in the geometry of the tissue to be irradiated is taken into account in the different irradiation scenarios.

4. Medical radiation apparatus according to one of claims 1 to 3,
**characterised in that**
visualisable characteristics of the radiation acting on the tissue are dosage distributions.

5. Medical radiation apparatus according to one of claims 1 to 4,
**characterised in that**
characteristics of the radiation acting on the tissue are visualised together with the tissue to be irradiated in a display (7).

6. Medical radiation apparatus according to one of claims 1 to 5,
**characterised in that**
the display (7) comprises the visualisation of the relevant geometric ranges of the radiation acting on the tissue.

7. Medical radiation apparatus according to one of claims 1 to 6,
**characterised in that**
visualisation of the characteristics of the radiation is provided for using the volume rendering method.

8. Medical radiation apparatus according to one of claims 1 to 7,
**characterised in that**
visualisation of the characteristics of the radiation is provided for using the surface rendering method.

9. Medical radiation apparatus according to one of claims 1 to 8,
**characterised in that**
layer by layer visualisation of the characteristics of the radiation is provided for.

## Revendications

1. Dispositif d'irradiation médical, comprenant une source de rayonnement (2), un système de déviation (3) et un dispositif de traitement de données (5), la source de rayonnement (2) et le système de déviation (3) pouvant être commandés au moyen du dispositif de traitement de données (5) en fonction d'un plan d'irradiation généré en utilisant une prise de vue, établie avec un appareil de diagnostic (6) médical générateur d'images, d'un tissu à irradier,
**caractérisé en ce que** le dispositif de traitement de données (5) est conçu au niveau de l'informatique de telle sorte que des caractéristiques de l'irradiation agissant sur le tissu peuvent être visualisées dans une représentation (7) commune, les caractéristiques étant déterminées sur la base d'au moins deux plans d'irradiation différents, à savoir un plan d'irradiation spécifique au passé et un plan d'irradiation spécifique à l'avenir, et les différents plans d'irradiation comportant différentes géométries du tissu à irradier.

2. Dispositif d'irradiation médical selon la revendication 1,
**caractérisé en ce que** la source de rayonnement (2) est une source à faisceaux de particules.

3. Dispositif d'irradiation médical selon la revendication 1 ou 2,
**caractérisé en ce qu'**une variation dans le temps de la géométrie du tissu à irradier est prise en compte dans les différents plans d'irradiation.

4. Dispositif d'irradiation médical selon l'une quelconque des revendications 1 à 3,
**caractérisé en ce que** des caractéristiques visualisables du rayonnement agissant sur le tissu sont des répartitions de dose.

5. Dispositif d'irradiation médical selon l'une quelconque des revendications 1 à 4,
**caractérisé en ce que** des caractéristiques du rayonnement agissant sur le tissu sont visualisées en même temps que le tissu à irradier dans une représentation (7).

6. Dispositif d'irradiation médical selon l'une quelconque des revendications 1 à 5,
**caractérisé en ce que** la représentation (7) comporte la visualisation des rayons d'action géométriques importants du rayonnement agissant sur le tissu.

7. Dispositif d'irradiation médical selon l'une quelconque des revendications 1 à 6,
**caractérisé en ce qu'**une visualisation des caractéristiques du rayonnement est prévue en utilisant le procédé volume rendering.

8. Dispositif d'irradiation médical selon l'une quelconque des revendications 1 à 7,
**caractérisé en ce qu'**une visualisation des caractéristiques du rayonnement est prévue en utilisant le procédé surface rendering.

9. Dispositif d'irradiation médical selon l'une quelconque des revendications 1 à 8,
**caractérisé en ce qu'**il est prévu une visualisation couche par couche des caractéristiques du rayonnement.
